# EUROPEAN PATENT APPLICATION

(11) **EP 4 830 725 A1**
(43) Date of publication of application: **28.10.2026**
(21) Application number: 25744750.8
(22) Date of filing: 24.01.2025
(51) Int. Cl.: A61K 45/06, A61K 31/536, A61K 31/519, A61P 35/00, A61K 31/501, A61K 31/4985

(54) **PHARMACEUTICAL COMPOSITION COMPRISING PRMT5 INHIBITOR AND EGFR INHIBITOR**

(30) Priority: 26.01.2024 CN 202410116672; 04.03.2024 CN 202410245514
(71) Applicant: Apeiron Therapeutics (Hong Kong) Ltd., Hong Kong 999077 (HK)
(72) Inventor: MAO, Weifeng, Shanghai 201210 (CN); YAO, Bing, Shanghai 201210 (CN); ASWAD, Fred Jullien, Shanghai 201210 (CN); LI, Mingxi, Shanghai 201210 (CN); GU, Xiaohui, Shanghai 201210 (CN); JOSEPH, James David, Shanghai 201210 (CN)
(74) Representative: Bayramoglu et al.
(86) International application number: PCT/CN2025/074945
(87) International publication number: WO 2025/157287

(57) **Abstract**

A pharmaceutical composition is provided, including a protein arginine methyltransferase 5 (PRMT5) inhibitor and an epidermal growth factor receptor (EGFR) inhibitor. The pharmaceutical composition can be used to treat various cancers, including solid tumors. The combination product can be used to treat any number of diseases related to PRMT5 and/or EGFR.

## Description

### TECHNICAL FIELD

The present disclosure belongs to the field of medicine, and specifically relates to a pharmaceutical composition comprising a PRMT5 inhibitor and an EGFR inhibitor.

### BACKGROUND

Cancer is among the leading causes of death worldwide. Popular treatments, such as chemotherapy and immunotherapy, are limited in that their cytotoxic effects are not restricted to cancer cells and can also cause adverse side effects in normal tissues.

PRMT5 is a type II arginine methyltransferase that regulates important cellular functions, including cell cycle progression, apoptosis, and the DNA damage response, by symmetrically dimethylating proteins involved in transcription and signal transduction. However, data from genome-wide genetic perturbation screens using shRNA revealed a selective requirement for PRMT5 activity in MTAP-deleted cancer cell lines (Kruykov et al., 2016; Marjon et al., 2016, and Markarov et al., 2016). The accumulation of MTA caused by MTAP deletion in these cell lines partially inhibits PRMT5, making these cells selectively sensitive to additional PRMT5 inhibition.

Certain PRMT5 inhibitors have been developed, but they do not show selectivity for MTAP-deleted cancer cell lines. This lack of selectivity could be explained by the inhibitors' mechanism of action, as they are either SAM-uncompetitive or SAM-competitive inhibitors and therefore independent of MTAP (Kruykov et al., 2016; Marjon et al., 2016, and Markarov et al., 2016).

By using inhibitors that bind PRMT5 non-competitively or cooperatively with MTA, selectivity for MTAP-deleted/MTA-accumulating cells can be improved. PRMT5 inhibitors that bind non-competitively or cooperatively with MTA will exhibit increased binding to PRMT5 in the presence of MTA compared to the binding of the same inhibitor in the absence of MTA. Consequently, such inhibitors will bind with significantly greater potency in the presence of high concentrations of MTA and, therefore, lead to preferential inhibition of PRMT5 in MTA-accumulating cells relative to normal cells.

Recently, Thierry et al. reported that the first-generation PRMT5 inhibitors and EGFR inhibitors have a significant cooperative effect on the antiproliferative activity of triple-negative breast cancer cells. It provides a new and promising clinical treatment strategy for triple-negative breast cancer (Dovepress, 2023, https://doi.org/10.2147/BCTT.S430513); it is well known that second-generation PRMT5 inhibitors have better selectivity and safety than first-generation PRMT5 inhibitors, so the research on the combination of second-generation PRMT5 inhibitors and EGFR inhibitors and their indications is of great value.

### SUMMARY

In order to address the technical problem, the present disclosure provides a pharmaceutical composition comprising a PRMT5 inhibitor of Formula (I) as a first active ingredient and an EGFR inhibitor compound as a second active ingredient: 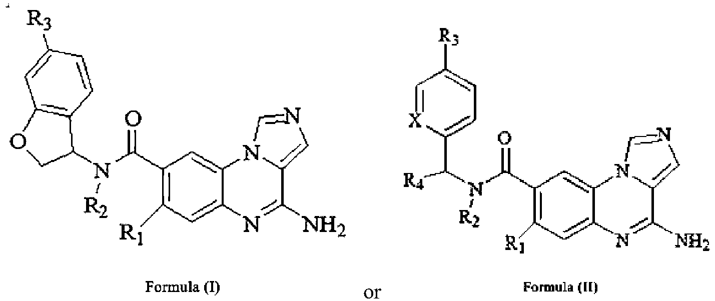 Wherein, in Formula (I) or Formula (II),
R₁ is H, halogen, C₁-C₆ alkyl, C₁-C₆ haloalkyl, and CN;
R₂ is H, C₁-C₆ alkyl, C₁-C₆ haloalkyl, and C₃-C₆ cycloalkyl;
R₃ is H, halogen, C₁-C₆ alkyl, C₁-C₆ haloalkyl, C₁-C₆ haloalkoxy, or SF₅;
in Formula II, R₄ is hydrogen or C₁-C₆ alkyl; X is CR₅ or N;
Wherein, R₅ is hydrogen, halogen, C₁-C₆ alkyl, C₁-C₆ haloalkyl, hydroxyl, -NH₂, or CN.

In one preferred embodiment of the present disclosure, wherein, in Formula (I) or Formula (II): :
R₁ is hydrogen, halogen, C₁-C₆ alkyl, and C₁-C₆ haloalkyl;
R₂ is hydrogen, C₁-C₆ alkyl, C₁-C₆ haloalkyl, and C₃-C₆ cycloalkyl;
R₃ is hydrogen, halogen, C₁-C₆ haloalkyl, C₁-C₆ haloalkoxy, or SF₅;
R₄ represents is hydrogen or methyl;
and in Formula II, X is CH or N.

In one preferred embodiment of the present disclosure, wherein, R₁ is hydrogen or fluorine.

In one preferred embodiment of the present disclosure, wherein, R₂ is cyclopropyl or methyl.

In one preferred embodiment of the present disclosure, wherein, R₃ is CF₃.

In one preferred embodiment of the present disclosure, wherein, R₄ is hydrogen or methyl.

In one preferred embodiment of the present disclosure, wherein, X is N.

In one preferred embodiment of the present disclosure, wherein, the PRMT5 inhibitor as the first active ingredient is selected from the group consisting of the following compounds and combinations thereof:

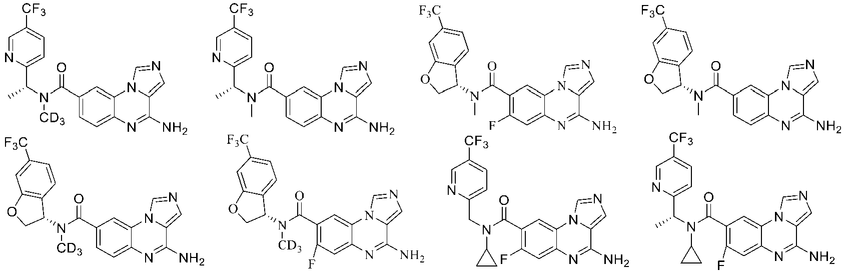

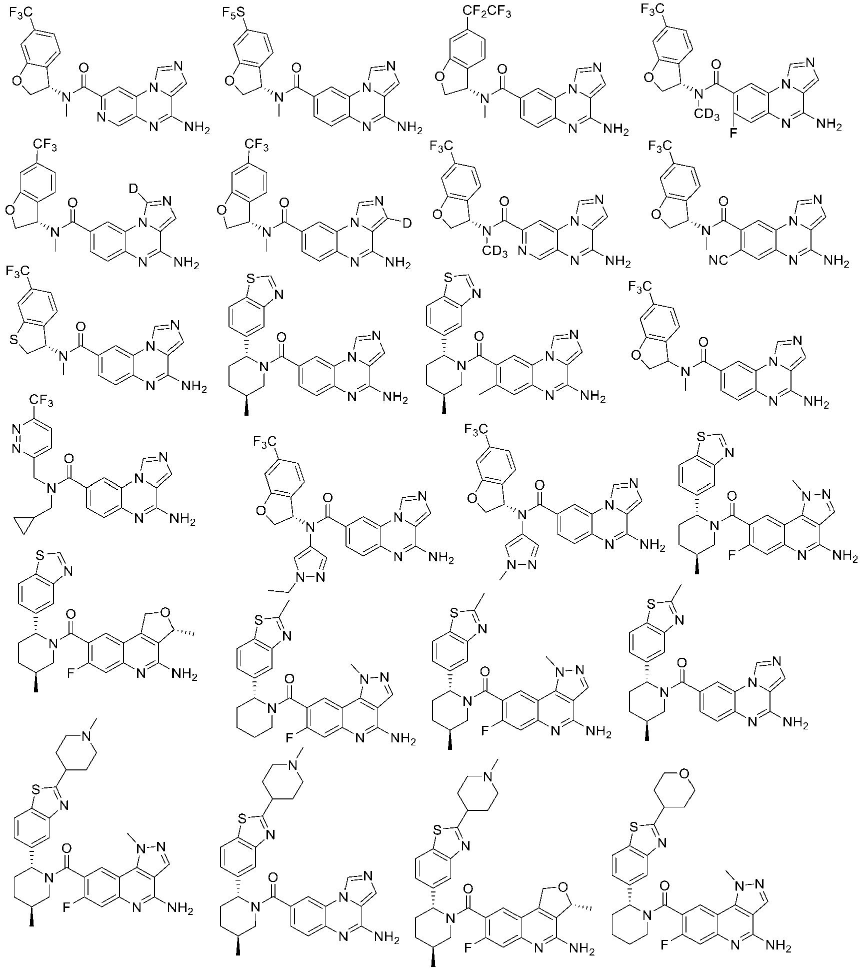

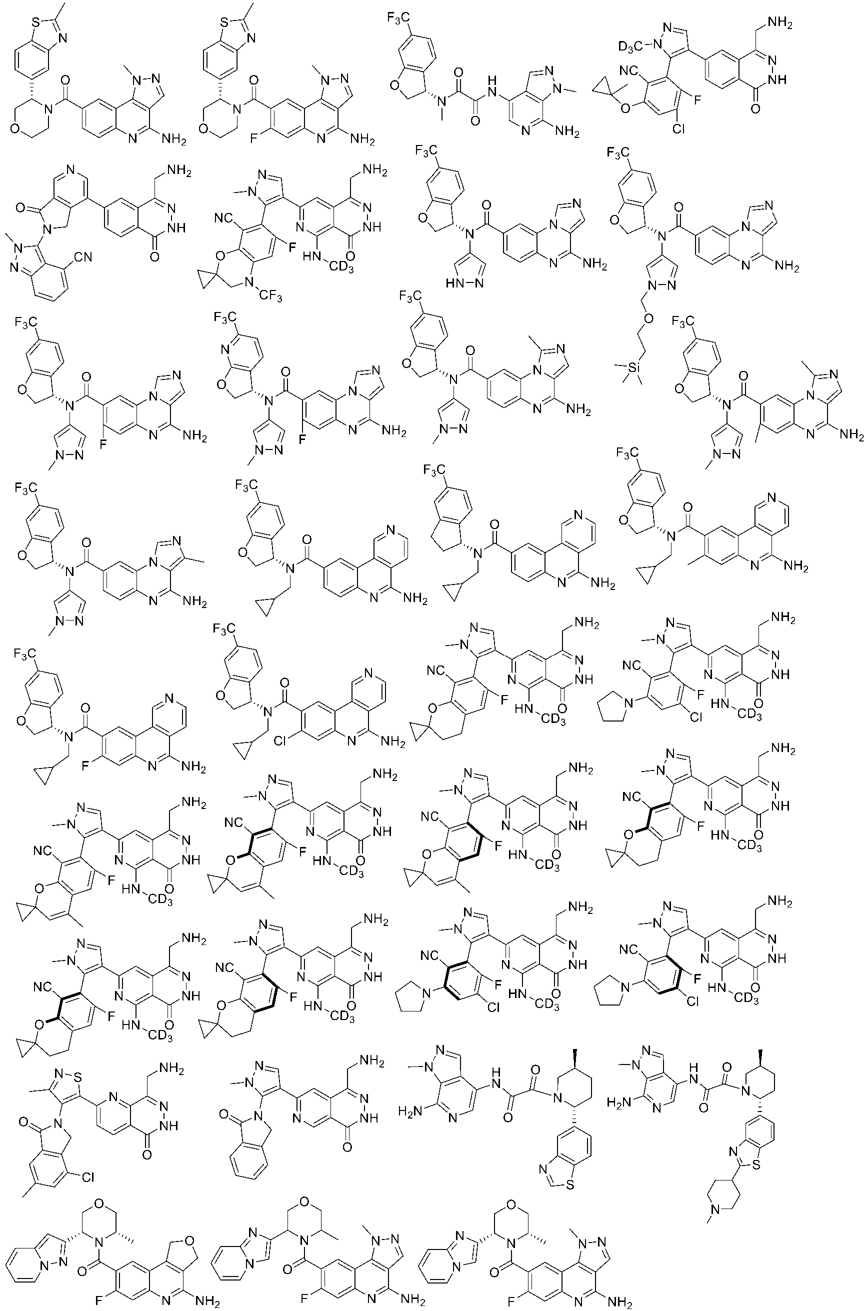

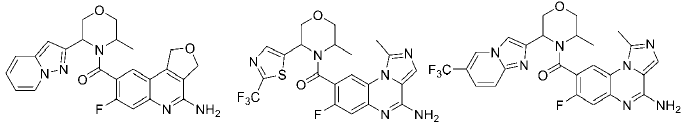

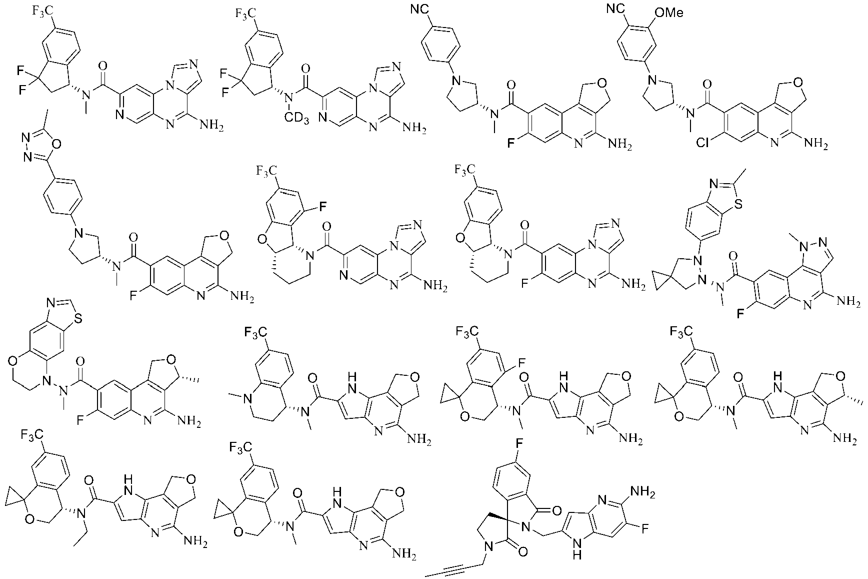

In one preferred embodiment of the present disclosure, wherein, the EGFR inhibitor as the second active ingredient is selected from any one of the following compounds or pharmaceutically acceptable salts thereof: Osimertinib (Tagrisso^{®}), Almonertinib (Ameile^{®}), Dacomitinib (VIZIMPRO^{®}), Afatinib (Gilotrif^{®}), Icotinib (Conmana^{®}), Erlotinib (Tarceva^{®}), Gefitinib (Iressa^{®}), Zorifertinib (AZD3759), Lazertinib, Nazartinib (EGF816), Rociletinib (CO1686), HM61713, Naquotinib (ASP8273), Mavelertinib (PF-06747775), Abivertinib (avitinib), Alflutinib (AST2818), Olafertinib (CX-101; RX-518), Almonertinib (aumolertinib; HS-10296), Rezivertinib (BPI-7711), Mobocertinib (TAK-788), BLU-945 (Bluprint), BLU-701 (Bluprint), BBT-176 (Bridge Biotherapeutics), TQB3804 (Chiatai Tianqing), BPI-361175 (Betta), QLH11811 (Qilu), HS10375 (Hansoh), H002 (Hongyun Biotech), DAJH-1050766 (Chengdu Diao Jiuhong), BI-4020 (Boehringer Ingelheim), CH7233163 (Chugai Pharmaceutical), JBI-09-063 or any combination thereof.

In one preferred embodiment of the present disclosure, wherein, the EGFR inhibitor as the second active ingredient is selected from Trastuzumab (Herceptin^{®}), Pertuzumab (Perjeta^{®}), Cetuximab (Erbitux^{®}), and Amivantamab (JNJ-6372).

In one preferred embodiment of the present disclosure, wherein, the EGFR inhibitor as the second active ingredient is selected from Osimertinib (Tagrisso^{®}), Almonertinib (Ameile^{®}), Dacomitinib (VIZIMPRO^{®}), Afatinib (Gilotrif^{®}), Icotinib (Conmana^{®}), Erlotinib (Tarceva^{®}), Gefitinib (Iressa^{®}) or pharmaceutically acceptable salts thereof and Trastuzumab (Herceptin^{®}), Pertuzumab (Perjeta^{®}), Cetuximab (Erbitux^{®}), and Amivantamab (JNJ-6372).

In another aspect, the present disclosure provides a method of treating cancer or a tumor, comprising administering the pharmaceutical composition of the present disclosure to a subject in need thereof.

In one preferred embodiment of the present disclosure, wherein, the tumor or cancer is selected from: glioblastoma multiforme, brain cancer, prostate cancer, pancreatic cancer, mantle cell lymphoma, non-Hodgkin's lymphoma and diffuse large B-cell lymphoma, acute myeloid leukemia, acute lymphoblastic leukemia, multiple myeloma, non-small cell lung cancer, small cell lung cancer, breast cancer, triple-negative breast cancer, gastric cancer, colorectal cancer, ovarian cancer, bladder cancer, hepatocellular carcinoma, esophageal cancer, bile duct cancer, mesothelioma, laryngeal cancer, melanoma, malignant peripheral nerve sheath tumor, osteosarcoma, myxoid chondrosarcoma, soft tissue sarcoma, oropharyngeal squamous cell carcinoma, chronic myeloid leukemia, epidermal squamous cell carcinoma, nasopharyngeal carcinoma, neuroblastoma, endometrial cancer, head and neck cancer, and cervical cancer.

In one preferred embodiment of the present disclosure, wherein, the tumor is non-small cell lung cancer.

In one preferred embodiment of the present disclosure, wherein, the non-small cell lung cancer is Osimertinib-resistant non-small cell lung cancer.

As will be understood by those of ordinary skill in the art, in any embodiment disclosed herein, any feasible combination of a compound of formula (I) or a pharmaceutically acceptable salt, solvate or prodrug thereof and a compound of formula (II) or a pharmaceutically acceptable salt, solvate or prodrug thereof is included in the present disclosure, as long as such combination is capable of producing some synergistic effect in the treatment of a subject in need of such treatment.

When any compound is used in the present disclosure, it includes any pharmaceutically acceptable form thereof, including but not limited to isomers, tautomers, salts, solvates, polymorphs, prodrugs, and so on. It should be understood that the term "compound" includes any and all such forms, whether or not explicitly stated, although sometimes only certain terms are explicitly stated, such as "salt" and "prodrug".

Unless expressly defined otherwise, all terms used herein have the ordinary meaning as would be interpreted or understood by one of ordinary skill in the art.

The terms "a", "an" or "the" used herein refer to both the singular and the plural forms. Generally, when a singular or plural form of a noun is used, it refers to both the singular and the plural forms of the noun.

When the term "about" is applied to a parameter, it means that the parameter can vary within +10%, preferably within ±5%, including any number from the lower limit to the upper limit. When the term "about" is applied to a range, it applies to both the lower and upper limits of the range. As will be understood by those skilled in the art, when a parameter is not critical, a number is generally given for illustrative purposes only and is not limited.

"Alkoxy" refers to a group -OR, wherein, R is alkyl as defined herein. Representative examples include methoxy, ethoxy, propoxy, isopropoxy, sec-butoxy, tert-butoxy, and the like.

"Alkyl" refers to a group derived from a straight or branched chain saturated hydrocarbon by removing a hydrogen from one of the saturated carbons. Alkyl groups preferably contain 1 to 8 carbon atoms, sometimes preferably 1 to 6 carbon atoms, and sometimes even more preferably 1 to 4 carbon atoms. Representative examples of alkyl groups include, but are not limited to, methyl, ethyl, n-propyl, isopropyl, n-butyl, sec-butyl, tert-butyl, pentyl, hexyl, and the like. "Lower alkyl," "lower alkoxy," or "lower haloalkyl" refers to an alkyl or alkyl moiety having one to four, sometimes preferably one to three or one to two carbon atoms.

As used herein, the term "cyano" refers to -CN.

The term "cycloalkyl" as used herein refers to a group derived from a monocyclic saturated carbocyclic ring by removing a hydrogen atom from the saturated carbocyclic ring, preferably having 3 to 8, more preferably 3 to 6 carbon atoms. Representative examples of cycloalkyl include, but are not limited to, cyclopropyl, cyclopentyl, and cyclohexyl.

As used herein, the terms "halo" and "halogen" refer to F, Cl, Br, or I.

The term "haloalkyl" as used herein refers to an alkyl group substituted with at least one halogen atom. A haloalkyl group can be an alkyl group in which all hydrogen atoms are substituted with halogens. Representative examples of haloalkyl groups include, but are not limited to, trifluoromethyl, fluoromethyl, difluoromethyl, bromomethyl, 1-chloroethyl, perchloroethyl, 2-fluoroethyl, and so on.

The term "heterocyclyl" as used herein refers to a 3 to 10-membered monocyclic or bicyclic non-aromatic group containing one or more, preferably 1 to 3, heteroatoms independently selected from nitrogen (N), oxygen and sulfur (S, S(O) or S(O)₂) in the non-aromatic ring. The heterocyclyl of the present disclosure can be connected to the parent molecular moiety through a carbon atom or a nitrogen atom in the group. The heterocyclyl group can be saturated or unsaturated, for example, containing one or more double bonds in the ring. Unless otherwise stated, the valence of the group can be located on any atom of any ring within the group where the valence rules permit. Examples may include, but are not limited to, azetidinyl, pyrrolidinyl, 2-oxopyrrolidinyl, 2,5-dihydro-1H-pyrrolyl, piperidinyl, 4-piperidinyl, morpholinyl, piperazinyl, 2-oxopiperazinyl, tetrahydropyranyl, tetrahydrofuranyl, 2-oxopiperidinyl, thiomorpholinyl, pyrazolidinyl, imidazolinyl, imidazolidinyl, and so on.

When any group, such as "cycloalkyl" or "heterocyclyl" is referred to as "substituted or unsubstituted" or "optionally substituted", unless otherwise specified, it means that the group is substituted or not substituted by 1 to 5, sometimes preferably 1 to 3 or 1 to 2 substituents independently selected from halogen, C₁-C₆ alkyl, C₁-C₆ alkoxy, C₁-C₆ haloalkyl and cyano.

The term "solvate" as used herein refers to a physical association of a compound of the invention with one or more, preferably one to three, solvent molecules (whether organic or inorganic). This physical association includes hydrogen bonding. In some cases, the solvate is capable of separation, for example when one or more, preferably one to three, solvent molecules are incorporated into the crystal lattice of a crystalline solid. Exemplary solvates include, but are not limited to, hydrates, ethanolates, methanolates, and isopropanolates. Solvation methods are generally known in the art.

"Prodrugs" refer to compounds that can be converted in vivo to produce the active parent compound under physiological conditions, such as by hydrolysis in the blood. Common examples include, but are not limited to, ester and amide forms of compounds having an active form with a carboxylic acid moiety. Amides and esters of the compounds of the present invention can be prepared according to conventional methods. In particular, in the present invention, prodrugs can also be formed by acylation of the amino group or nitrogen atom in the heterocyclyl ring structure, where the acyl group can hydrolyze in vivo. Such acyl groups include, but are not limited to, C₁-C₆ acyl groups, preferably C₁-C₄ acyl groups, more preferably C₁-C₂ (formyl or acetyl) groups, or benzoyl groups.

As used herein, the term "subject" refers to a human or other mammal, such as a monkey, dog, cat, or horse. The term is intended to encompass and is sometimes interchangeable with "patient".

As used herein, the terms "administering" or "drug administration" refer to providing a compound or pharmaceutical composition to a subject having or at risk for a disease or condition to be treated or prevented.

Any route of administration is suitable for the present disclosure. In one embodiment, the compounds of the present disclosure can be administered to a subject in a solid dosage form such as a tablet, capsule, or the like. In one embodiment, the compounds of the present disclosure can be administered to a subject by intravenous injection. In another embodiment, the compounds of the present disclosure can be administered to a subject by any other suitable systemic delivery method, such as oral, parenteral, intranasal, sublingual, rectal or transdermal administration.

As used herein, the term "therapeutically effective amount" refers to that amount of a compound or composition that will elicit the desired or intended biological or medical response in a subject that is being sought by a physician, veterinarian, or researcher. The therapeutically effective amount of the compound and the specific pharmaceutically acceptable carrier will vary depending on, for example, the age, weight, sex of the subject, the mode of administration, and the disease or condition being treated.

The term "pharmaceutically acceptable" when used before a compound, salt, prodrug, composition, or carrier means that such compound, salt, prodrug, composition, or carrier is suitable for administration to a subject for treatment without causing intolerable side effects to the subject considering the desired treatment.

As used herein, the term "pharmaceutically acceptable carrier" refers to a substance that is compatible with the compounds used in the present invention and can be used to administer the compounds in the methods of the present invention, and is preferably non-toxic, or inert and pharmaceutically acceptable. Pharmaceutically acceptable carriers can be solid, liquid or gaseous substances, including any and all dry powders, solvents, dispersion media, coatings, antibacterial and antifungal agents, isotonic agents, absorption delaying agents, and the like. Examples of such carriers include oils such as corn oil, buffers such as phosphate buffered saline (PBS), saline, polyethylene glycol, glycerol, polypropylene glycol, dimethyl sulfoxide, amides such as dimethylacetamide, proteins such as albumin, detergents such as Tween 80, monosaccharides and oligosaccharides such as glucose, lactose, cyclodextrins, starch, and the like.

As described herein, some embodiments of the compounds of the present disclosure can contain basic functional groups, such as amino or alkylamino, and therefore can form pharmaceutically acceptable salts with pharmaceutically acceptable acids. In this respect, the term "pharmaceutically acceptable salts" refers to relatively nontoxic inorganic and organic acid addition salts of the compounds of the present disclosure. These salts can be prepared on site during the administration of carriers or dosage form production processes, or by reacting the purified compounds of the present invention in free alkali form with suitable organic or inorganic acids alone, and separating the salts so formed in subsequent purification processes to prepare. Representative salts include hydrobromide, hydrochloride, sulfate, bisulfate, phosphate, nitrate, acetate, valerate, oleate, palmitate, stearate, laurate, benzoate, lactate, phosphate, toluenesulfonate, citrate, maleate, fumarate, and succinate.

The formulations used in the present disclosure may also contain stabilizers, preservatives, buffers, antioxidants or other additives known to those skilled in the art. The use of such media and agents for pharmaceutically active substances is well known in the art.

The terms "synergistic" and the like as used herein refer to an effect caused by a combination of two or more agents that is greater than the cumulative effect of the two or more agents used alone. This synergistic effect of combination therapy includes higher efficacy, lower side effects, or both. In some embodiments, the synergistic effect includes a significant reduction in the side effects of the two therapeutic inhibitors due to a reduction in the dosage of the two therapeutic inhibitors, while the overall therapeutic efficacy remains at approximately the same or improved levels. In some embodiments, the synergistic effect includes a significant improvement in the efficacy of inhibiting cancer cell proliferation, while the side effects caused by the two drugs remain at approximately the same or lower levels. The synergistic effect allows the use of a lower dose of a single drug to effectively treat the disease. In general, the synergistic combination of two or more drugs can lead to improvements in disease treatment compared to monotherapy.

Combination therapy can allow the use of lower doses of a first therapeutic agent, such as a PRMT5 inhibitor, or a second therapeutic agent, such as an EGFR inhibitor, or lower doses of both therapeutic agents than would normally be required when either agent is used alone. The present disclosure encompasses any and all such "synergistic" effects.

The pharmaceutical composition may contain a PRMT5 inhibitor and an EGFR inhibitor for use in the methods of the present disclosure in an amount ranging from 0.01% to 99% by weight of the total composition, preferably from 0.1% to 80% by weight of the total composition, and more preferably from 0.1% to 50% by weight of the total composition. The weight ratio between the PRMT5 inhibitor and the EGFR inhibitor may be in the range of 1:20 to 20:1, sometimes preferably from 1:15 to 15:1, and sometimes more preferably from 1:10 to 10:1.

For systemic administration, the daily dosage range for adult human treatment of a PRMT5 inhibitor is about 0.01 to about 150 mg/kg, preferably about 0.05 to about 100 mg/kg, and sometimes more preferably about 0.1 to about 50 mg/kg.

The present disclosure provides pharmaceutical compositions that can be used to treat and/or prevent various cancers that may include or exclude the following cancers: glioblastoma multiforme, brain cancer, prostate cancer, pancreatic cancer, mantle cell lymphoma, non-Hodgkin lymphoma and diffuse large B-cell lymphoma, acute myeloid leukemia, acute lymphoblastic leukemia, multiple myeloma, non-small cell lung cancer, small cell lung cancer, breast cancer, triple-negative breast cancer, gastric cancer, colorectal cancer, ovarian cancer, bladder cancer, hepatocellular carcinoma, esophageal cancer, bile duct cancer, mesothelioma, laryngeal cancer, melanoma, malignant peripheral nerve sheath tumor, osteosarcoma, myxoid chondrosarcoma, soft tissue sarcoma, oropharyngeal squamous cell carcinoma, chronic myeloid leukemia, epidermal squamous cell carcinoma, nasopharyngeal carcinoma, neuroblastoma, endometrial cancer, head and neck cancer, and cervical cancer.

In one preferred embodiment of the present disclosure, wherein, the tumor is non-small cell lung cancer.

In one preferred embodiment of the present disclosure, wherein, the non-small cell lung cancer is Osimertinib-resistant non-small cell lung cancer.

In one preferred embodiment of the present disclosure, wherein, the cancer is metastatic cancer.

In one preferred embodiment of the present disclosure, wherein, the metastatic cancer is brain metastatic cancer.

### BRIEF DESCRIPTION OF DRAWINGS

Figure 1A-1B: Dose-response curves and percent growth inhibition of Compound A, osimertinib, and their combination in the NCI-H292 MTAP-deficient cell model
Figure 2A-2B: Dose-response curves and percent growth inhibition of Compound C, osimertinib, and their combination in the NCI-H292 MTAP-deficient cell model
Figure 3: In vivo antitumor efficacy of Compound A, osimertinib, and their combination in an osimertinib-resistant NSCLC PDX model in NU/NU mice

### DETAILED DESCRIPTION OF THE EMBODIMENTS

### 1. Experimental compounds

The compound Osimertinib used for tests is a drug developed by AstraZeneca, with the trade name Tagrisso, which has the following chemical structure:

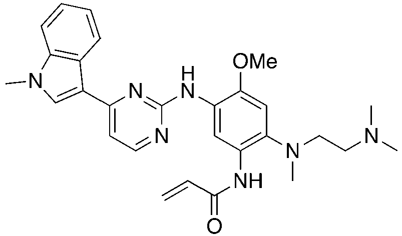

The chemical name of compound A used in this test is (S)-4-amino-N-methyl-N-(6-(trifluoromethyl)-2,3-dihydrobenzofuran-3-yl)imidazo[1,5-a]quinoxaline-8-carboxamide, which has the following chemical structure:

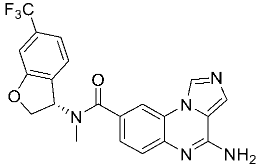

The chemical name of compound B used in this test is: 4-amino-N-cyclopropyl-7-fluoro-N-(5-(trifluoromethyl)pyridin-2-yl)methyl)imidazo[1,5-a]quinoxaline-8-carboxamide, which has the following chemical structure:

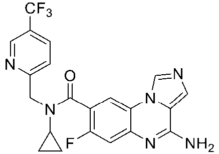

The chemical name of compound C used in this test is: (R)-4-amino-N-cyclopropyl-7-fluoro-*N*-(1-(5-trifluoromethyl)pyridin-2-yl)ethyl)imidazo[1,5-a]quinoxaline-8-carboxamide, which has the following chemical structure:

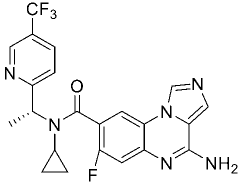

The chemical name of compound D used in this test is: (S)-4-amino-7-fluoro-N-methyl-N-(6-trifluoromethyl)-2,3-dihydrobenzofuran-3-yl)imidazo[1,5-a]quinoxaline-8-carboxamide, which has the following chemical structure:

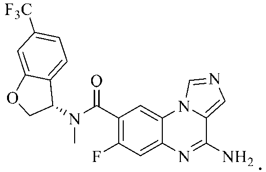

The chemical name of compound E used in this test is: (S)-4-amino-N-methyl-N-(6-(trifluoromethyl)-2,3-dihydrobenzofuran-3-yl)imidazo[1,5-a]pyrido[3,4-e]pyrazine-8-carboxamide, which has the following chemical structure:

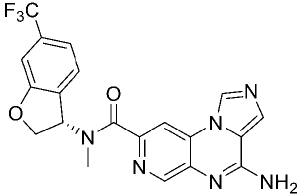

The chemical name of compound F used in this test is: (S)-4-amino-N-(methyl-d₃)-N-(6-(trifluoromethyl)-2,3-dihydrobenzofuran-3-yl)imidazo[1,5-a]quinoxaline-8-carboxamide, which has the following chemical structure:

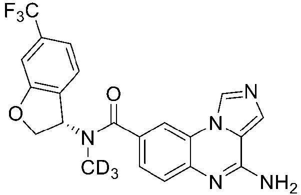

The chemical name of compound G used in this test is: (S)-4-amino-N-methyl-N-(6-(pentafluoro-λ⁶-sulfane)-2,3-dihydrobenzofuran-3-yl)imidazo[1,5-a]quinoxaline-8-carboxamide, which has the following chemical structure:

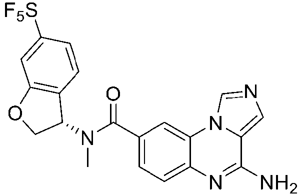

The chemical name of compound H used in this test is: (S)-4-amino-N-methyl-N-(6-(perfluoroethyl)-2,3-dihydrobenzofuran-3-yl)imidazo[1,5-a]quinoxaline-8-carboxamide, which has the following chemical structure:

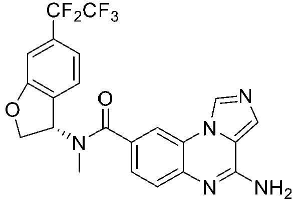

The chemical name of compound I used in this test is: (S)-4-amino-7-fluoro-N-(methyl-d₃)-N-(6-(trifluoromethyl)-2,3-dihydrobenzofuran-3-yl)imidazo[1,5-a]quinoxaline-8-carboxamide, which has the following chemical structure:

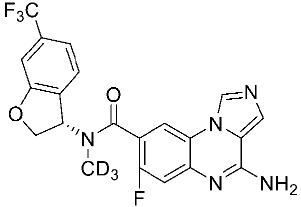

The chemical name of compound J used in this test is: (S)-4-amino-N-methyl-N-(6-(trifluoromethyl)-2,3-dihydrobenzofuran-3-yl)imidazo[1,5-a]quinoxaline-8-carboxamide-1-*d*, which has the following chemical structure:

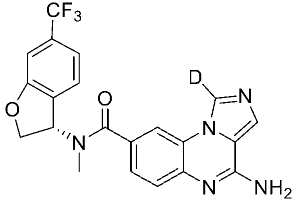

The chemical name of compound K used in this test is (S)-4-amino-N-methyl-N-(6-(trifluoromethyl)-2,3-dihydrobenzofuran-3-yl)imidazo[1,5-a]quinoxaline-8-carboxamide-3-*d*, which has the following chemical structure:

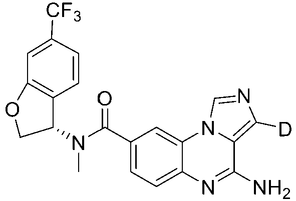

The chemical name of compound L used in this test is: (S)-4-amino-N-(methyl-d₃)-N-(6-(trifluoromethyl)-2,3-dihydrobenzofuran-3-yl)imidazo[1,5-a]pyrido[3,4-e]pyrazine-8-carboxamide, which has the following chemical structure:

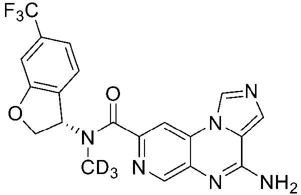

The chemical name of the compound M used in this test is: (S)-4-amino-7-cyano-N-methyl-N-(6-(trifluoromethyl)-2,3-dihydrobenzofuran-3-yl)imidazo[1,5-a]quinoxaline-8-carboxamide, which has the following chemical structure:

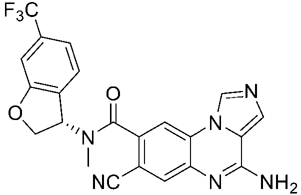

The chemical name of the compound N used in this test is: (R)-4-amino-N-(methyl-d₃)-N-(1-(5-(trifluoromethyl)pyridin-2-yl)ethyl)imidazo[1,5-a]quinoxaline-8-carboxamide, which has the following chemical structure:

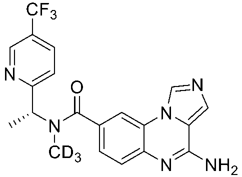

The chemical name of compound O used in this test is: (R)-4-amino-N-methyl-N-(1-(5-(trifluoromethyl)pyridin-2-yl)ethyl)imidazo[1,5-a]quinoxaline-8-carboxamide, which has the following chemical structure:

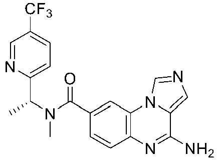

The preparation of Compounds A, D, E, F, G, H, I, J, K, L, and M is described in international patent application PCT/CN2023/120923, and the preparation of Compounds B, C, N, and O is described in international patent application PCT/CN2023/111604, the entirety of each of which is incorporated herein by reference.2. Combination of compounds in the NCI-H292 MTAP deleted cell model

### Cell lines and cell culture:

NCI-H292 MTAP deleted cell line was purchased from Nanjing Cobioer Biotechnology Co., Ltd. The cells were cultured in a cell culture medium containing 90% RPMI1640 and 10% fetal bovine serum at 37°C and 5% CO₂. Experiments could only be performed on cells in the logarithmic growth phase.

### Combination drug testing:

Adjust the cell density in complete medium and seed the cells into 96-well culture plates. Incubate the plates at 37 °C with 5% CO₂. Add the two test compounds to the plates using a serial dilution scheme. Continue incubation at 37 °C and 5% CO₂ for 144 h. Thaw the CellTiter-Glo (CTG) reagent and equilibrate the plates to room temperature for ~30 min. Add an equal volume of CTG reagent to each well, shake on an orbital shaker for 5 min to lyse the cells, and allow the signal to stabilize at room temperature for 20 min. Measure luminescence on a plate reader and record the data. Data analysis: 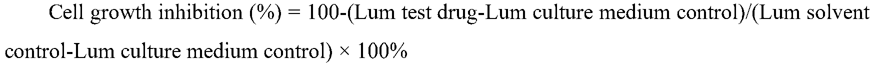 Cell growth inhibition (%) = 100-(Lum test drug-Lum culture medium control)/(Lum solvent control-Lum culture medium control) × 100%

Calcusyn was used to analyze the combination index (CI) values.

### Example 1: Single-agent evaluation of compounds in the NCI-H292 MTAP-deficient cell model.

**Table 1**

| **Compound name** | **Max Inh%** | **IC₂₀ (nM)** | **IC₅₀ (nM)** |
|---|---|---|---|
| **Compound A** | 71.2 | 10.7 | 117 |
| **Compound C** | 64.6 | 9.60 | 49.9 |
| **Osimertinib** | 91.1 | 18.6 | 50.2 |

Table 1 reports the IC₂₀ and IC₅₀ values of each compound in the NCI-H292 MTAP deleted cells, as well as the maximum percent inhibition.

### Example 2: Study on the test of combination of compound A and Osimertinib in the NCI-H292 MTAP-deleted cell model

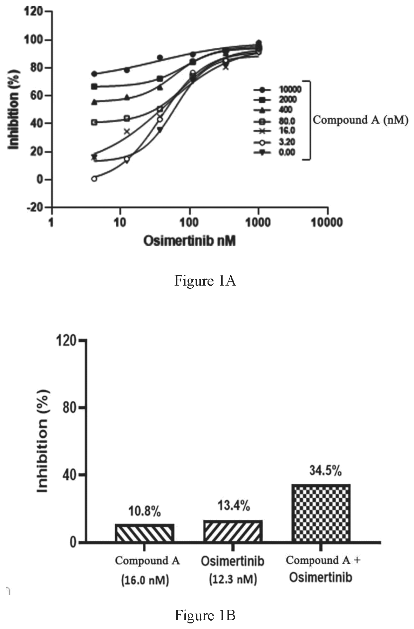

The results of the combined use of compound A and Docetaxel on NCI-H292 MTAP deleted cells are shown in Table 2 for inhibition analysis, Figure 1A for inhibition curves, and Figure 1B for inhibition rates:

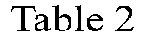

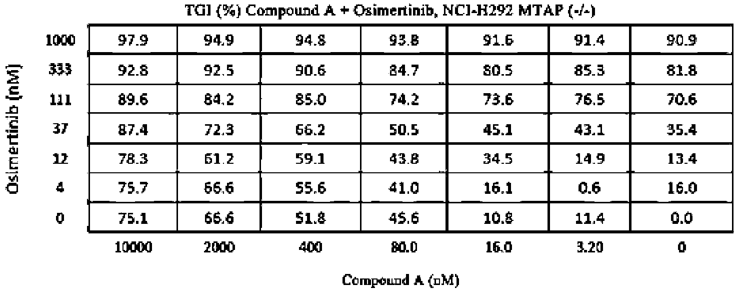

Figure 1B reports the inhibition rates of compound A (16.0 nM) and Osimertinib (12.3 nM) alone and in combination, indicating that under this condition, the two compounds exhibited a synergistic effect.

The analysis of the combination index (CI) is shown in Table 3:

**Table 3**

| | Compound A + Osimertinib CI, NCI-H292 MTAP (-/-) | | | | | | **Osimertinib [nM]** |
|---|---|---|---|---|---|---|---|
| | **0.33** | **0.63** | **0.63** | **0.74** | **1.02** | **1.05** | **1000.0** |
| | **0.38** | **0.32** | **0.39** | **0.71** | **0.99** | **0.67** | **333.3** |
| | **0.34** | **0.35** | **0.25** | **0.52** | **0.52** | **0.44** | **111.1** |
| | **0.36** | **0.67** | **0.45** | **0.76** | **0.84** | **0.88** | **37.0** |
| | **1.23** | **1.60** | **0.49** | **0.58** | **0.61** | **2.25** | **12.3** |
| | **1.64** | **0.91** | **0.55** | **0.50** | **2.11** | **1341** | **4.1** |
| **Compound A (nM)** | **10000** | **2000** | **400.0** | **80.0** | **16.0** | **3.20** | |

Table 3 exhibits the combined effect index CI value of compound A and Osimertinib.

### Example 3: Study on the test of combination of compound C and Osimertinib in the NCI-H292 MTAP-deleted cell model

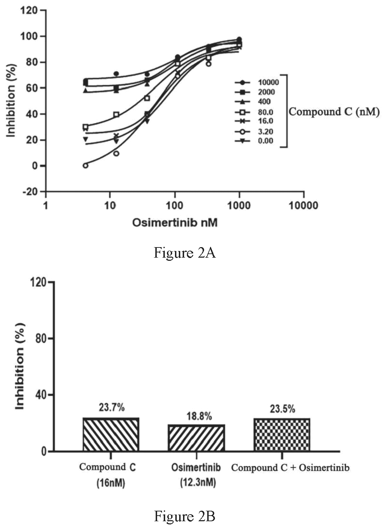

The results of the combined use of compound C and Docetaxel on the NCI-H292 MTAP deleted cell model are shown in Table 4 for inhibition analysis, Figure 2A for inhibition curves, and Figure 2B for inhibition rates:

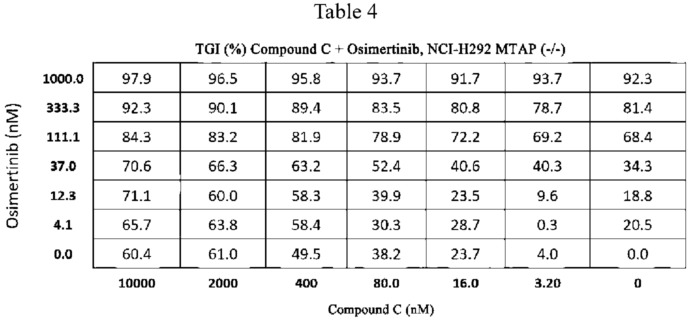

Figure 2B reports the inhibition rates of compound C (16.0 nM) and Osimertinib (12.3 nM) alone and in combination, indicating that under this condition, the two compounds exhibited an additive effect.

Analysis of the combination index (CI)

**Table 5**

| | Compound C + Osimertinib CI, NCI-H292 MTAP (-/-) | | | | | | **Osimertinib (nM)** |
|---|---|---|---|---|---|---|---|
| | 0.28 | 0.40 | 0.47 | 0.70 | 0.95 | 0.70 | 1000.0 |
| | 0.34 | 0.41 | 0.43 | 0.79 | 0.99 | 1.16 | 333.3 |
| | 0.48 | 0.32 | 0.32 | 0.39 | 0.61 | 0.74 | 111.1 |
| | 1.64 | 0.74 | 0.47 | 0.71 | 1.25 | 1.24 | 37.0 |
| | 1.42 | 0.99 | 0.35 | 0.66 | 1.50 | 6.00 | 12.3 |
| | 2.43 | 0.62 | 0.25 | 0.91 | 0.43 | 4024 | 4.1 |
| Compound C (nM) | **10000** | **2000** | **400.0** | **80.0** | **16.0** | **3.20** | |

Table 5 exhibits the combined effect index CI value of compound C and Osimertinib.

Conclusion: The combination index of compound A and compound C with the EGFR inhibitor Osimertinib ranged from 0.32 to 2.23, showing an additive effect.

### 3. Combination of the compound in in the PDX NU/NU mouse model of Osimertinib-resistant non-small cell lung cancer

### Example 1: In vivo pharmacodynamic study of the compound in the PDX NU/NU mouse model of Osimertinib-resistant non-small cell lung cancer

### Animal modeling and grouping:

NU/NU mice, female, 6-8 weeks old, weighing approximately 18-22 g. They were purchased from Beijing Vital River Laboratory Animal Technology Co., Ltd. All experimental mice were kept in the SPF-level animal room of Xi'an LIDE and acclimated for at least 3 days in advance. The lung cancer model tumor-bearing mice were chosen. When a tumor grew to 800-1000 mm³, the tumor was taken out under sterile conditions and passed it subcutaneously into NU/NU mice. When the average tumor volume reached about 150 mm³ (100-200 mm³), 18 tumor-bearing mice were chosen and randomly divided into 6 groups according to the tumor volume. The day of grouping was Day 0, and they were administered according to the following solution:

**Table 6. Administration and grouping**

| **Group** | **Administration** | **N** | **Dose** | **Frequency of administration** | **Route of administr ation** |
|---|---|---|---|---|---|
| | | | | | |
| Group 1 | Control group | 3 | -- | QD+QD×56 days | PO+PO |
| Group 2 | Osimertinib | 3 | 10 | QD×56 days | PO |
| Group 3 | Compound A | 3 | 10 | QD×56 days | PO |
| Group 4 | Osimertinib + compound A | 3 | 10+10 | QD+QD×56 days | PO+PO |

| | | | | | |
|---|---|---|---|---|---|
| N: Number of animals; dose volume: 10 µL/g; PO: per os. Administer Osimertinib or Osimertinib solvent first, and administer compound A or compound A solvent after an interval of 1 hour. | | | | | |

**Table 7. PDX model information**

| **Model No.** | **Type of cancer** | **Patient gender** | **Age** | **Pathological diagnosis** |
|---|---|---|---|---|
| LD1-0025-200729 | Lung cancer | Female | 39 | Poorly differentiated adenocarcinoma |

### Animal housing:

All mice were housed in an SPF-level animal room IVC constant temperature and pressure system with a temperature of 20~26°C, humidity of 40~70%, and a light cycle of 12 hours on and 12 hours off. No more than 5 mice were kept in each cage. The bedding used in the cage was autoclaved corn cobs, which were replaced 1-2 times a week. During the entire experiment, all experimental mice had free access to food and drink, feed was sterilized by Co60 irradiation, drinking water was autoclaved, and both feed and drinking water were kept in sufficient supply. All personnel entering and leaving the animal feeding room or experimental operators wore sterilized laboratory clothes, disposable medical masks, and rubber gloves. Each cage had a corresponding clear and detailed label, which includes: number of animals, gender, strain, receipt date, project number, group, current experimental stage, and experimental person in charge. Animals were numbered using a mouse ear punch. All laboratory animals were acclimated for 3 days before use in the experiment.

### Aseptic operation:

The test compound was prepared for use in a biosafety cabinet. All operations during the experiment, including drug administration, measurement of tumor volume and weight, were completed in the biosafety cabinet in the animal room.

### Grouping design:

All tumor-bearing mice were measured for tumor volume and weighed before grouping. Tumor-bearing mice were randomized according to the measured tumor volume. The principle of random grouping design was adopted. First, the mice were divided into groups according to tumor volume, and then the mice in each group were randomly assigned to each treatment team. This method was used to reduce system errors.

### Experimental observations:

The experimental plan and the use of laboratory animals in the experiment were reviewed, discussed, revised and approved by the LIDE Biological IACUC. Throughout the experiment, the use and observation of laboratory animals were carried out in accordance with AAALAC regulations. After the laboratory animals were inoculated with tumor tissue, they were observed every day to record their morbidity, death, and so on. During routine experiments, all laboratory animals were monitored for behavior, food intake, water intake, weight changes, and other abnormal conditions. Evaluation indicators:

The main purpose was to detect the growth inhibition effect or complete cure ability of the test drug compound A as a single drug or in combination with Osimertinib on the human lung cancer PDX model LD1-0025-200729.
- Tumor volume and weight measurement of tumor-bearing mice: A vernier caliper was used to measure twice a week. The calculation equation for tumor volume is V = 0.5 a × b², where a and b represent the major and minor diameters of the tumor, respectively;
- Tumor growth inhibition: TGI (%) = [1-(Tᵢ-T₀)/ (Vᵢ-V₀)] ×100
   Tᵢ is the mean tumor volume after the start of administration in the compound group, T₀ is the mean tumor volume at the first dose in the compound group, V₀ is the mean tumor volume at the first dose in the vehicle control group, and Vᵢ is the mean tumor volume after the start of administration in the vehicle control group.
- Relative tumor proliferation rate T/C (%): The calculation equation is as follows: T/C% = T_{RTV} / C_{RTV} × 100% (T_{RTV}: treatment group RTV; C_{RTV}: negative control group RTV). The relative tumor volume (RTV) was calculated based on the results of tumor measurement, and the calculation equation is RTV = Vₜ / V₀, where V₀ is the average tumor volume measured at the time of group administration (i.e. d₀), Vₜ is the average tumor volume at a certain measurement, and T_{RTV} and C_{RTV} were taken from the same day data.
- The weight of all tumor-bearing mice was measured once a day, and the weight of mice was measured at the same time every day within a 2-hour window. At the same time, the rate of change in weight gain of mice after administration was calculated: RCBW (%) = (BWᵢ - BW₀)/BW₀×100, where BWᵢ is the weight after the start of administration and BW₀ is the weight at the first dose.
- After the experiment, the tumor mass was weighed and photographed.

### Experiment termination:

When individual tumor-bearing mice were extremely emaciated and on the verge of death or their tumor volume reached 2,500 mm³ in the experiment, they were euthanized in advance. Safety evaluation of test drug:

During the experiment, when the weight of treated mice continued to decrease and RCBW reached more than 15%, the dosage was adjusted. The specific adjustment range depended on the status of tumor-bearing mice and the adjustment was performed after negotiation. At the same time, the behavior, fur and other abnormal manifestations of tumor-bearing mice were observed and recorded during treatment, and reported to the project leader in a timely manner. When RCBW reached more than 20%, the treated mice were considered to be discontinued for observation until their status recovered and RCBW was less than 10%.

### Experimental data analysis:

All data were expressed as Mean + SEM and statistically analyzed by one-way ANOVA using the statistical analysis software Graphpad10. *P < 0.05 was considered a statistically significant difference. The test results are shown in Table 8.

**Table 8: Statistics of tumor volume data**

| Administration time (days) | Tumor volume (mm³) | | | | | | | |
|---|---|---|---|---|---|---|---|---|
| | Group 1 | | Group 2 | | Group 3 | | Group 4 | |
| | Mean | SEM | Mean | SEM | Mean | SEM | Mean | SEM |
| 0 | 155.2 | 18.9 | 155.7 | 27.2 | 155.2 | 17.9 | 155.2 | 24.8 |
| 4 | 186.3 | 22.6 | 185.9 | 33.3 | 179.8 | 19.6 | 179.7 | 28.1 |
| 7 | 220.2 | 38.3 | 208.3 | 41.6 | 205.2 | 23.6 | 220.6 | 38.6 |
| 11 | 253.0 | 42.0 | 232.2 | 51.7 | 228.6 | 42.7 | 252.8 | 47.2 |
| 14 | 281.6 | 47.7 | 253.6 | 44.1 | 251.0 | 38.9 | 278.1 | 58.0 |
| 18 | 316.9 | 44.4 | 288.4 | 45.0 | 274.5 | 32.3 | 284.3 | 68.4 |
| 21 | 353.0 | 48.3 | 310.4 | 50.8 | 271.3 | 29.9 | 271.8 | 54.3 |
| 25 | 452.1 | 52.3 | 369.2 | 73.4 | 315.2 | 32.9 | 268.4 | 59.9 |
| 28 | 491.0 | 50.2 | 407.1 | 73.6 | 321.2 | 38.2 | 259.8 | 32.8 |
| 32 | 549.6 | 56.9 | 439.2 | 83.9 | 318.3 | 29.5 | 216.3 | 14.5 |
| 35 | 620.7 | 62.4 | 470.2 | 99.1 | 308.3 | 52.2 | 200.9 | 8.5 |
| 39 | 700.6 | 82.9 | 501.3 | 125.0 | 288.3 | 45.5 | 186.4 | 14.3 |
| 42 | 764.6 | 75.1 | 534.5 | 135.5 | 266.9 | 35.6 | 174.2 | 12.5 |
| 46 | 810.9 | 96.8 | 589.4 | 179.9 | 278.7 | 40.3 | 147.4 | 21.2 |
| 49 | 880.5 | 99.7 | 605.3 | 190.3 | 281.0 | 43.6 | 140.2 | 20.7 |
| 53 | 981.9 | 109.6 | 633.7 | 181.3 | 283.7 | 48.9 | 126.5 | 24.6 |
| 56 | 1076.2 | 116.3 | 679.4 | 197.1 | 287.7 | 48.5 | 110.2 | 26.0 |
| 57 | 1093.4 | 116.0 | 689.6 | 199.1 | 288.1 | 46.8 | 109.4 | 25.9 |

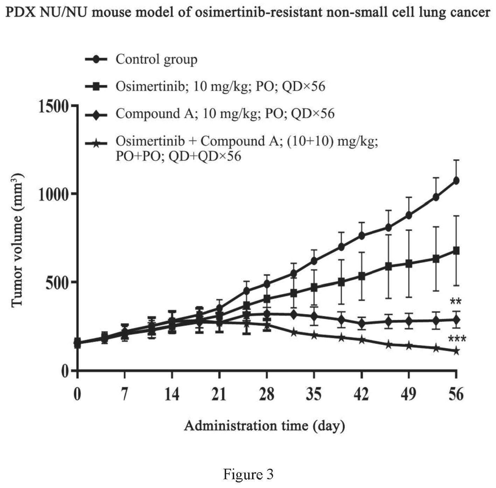

In the 56-day in vivo pharmacodynamic study of the PDX NU/NU mouse model of Osimertinib-resistant non-small cell lung cancer, the changes in tumor volume of each group of animals during administration are detailed in Table 8 and Figure 3. The tumor growth inhibition (TGI) of the Osimertinib single-drug group (10 mg/kg, PO, once daily) was 43.1%; the TGI of the compound A single-drug group (10 mg/kg, PO, once daily) was 85.8% (**P<0.01); the tumor growth inhibition (TGI) of the compound A (10 mg/kg, PO, once daily) combined with Osimertinib (10 mg/kg, PO, once daily) group reached 104.9% (***P<0.001).

The results showed that there was no statistically significant difference in the Osimertinib single drug group compared with the control group; there was a statistically significant difference in the compound A single drug group compared with the control group; there was a statistically significant difference in the combination of compound A and Osimertinib compared with the control group. Compared with the single drug group, the combination of compound A and Osimertinib showed stronger antitumor activity.

Although preferred examples have been described hereinabove, it will be apparent to those skilled in the art that modifications may be made without departing from the present disclosure. Such modifications are considered to be possible variants encompassed within the scope of the present disclosure.

## Claims

1. A pharmaceutical composition comprising a PRMT5 inhibitor of Formula (I) or Formula (II) as a first active ingredient and an EGFR inhibitor compound as a second active ingredient: 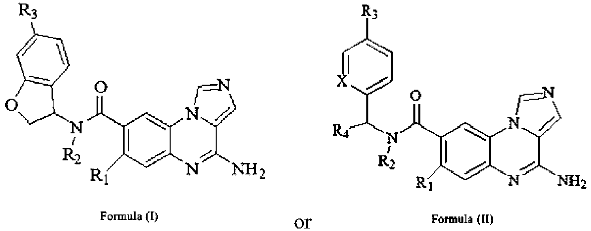 wherein, in Formula (I) or Formula (II),
R₁ is H, halogen, C₁-C₆ alkyl, C₁-C₆ haloalkyl, and CN;
R₂ is H, C₁-C₆ alkyl, C₁-C₆ haloalkyl, and C₃-C₆ cycloalkyl;
R₃ is H, halogen, C₁-C₆ alkyl, C₁-C₆ haloalkyl, C₁-C₆ haloalkoxy, or SF₅;
in Formula II, R₄ is hydrogen or C₁-C₆ alkyl; X is CR₅ or N;
wherein, R₅ is hydrogen, halogen, C₁-C₆ alkyl, C₁-C₆ haloalkyl, hydroxyl, -NH₂, or CN.

2. The pharmaceutical composition according to claim 1, wherein, in Formula (I) or Formula (II):
R₁ is hydrogen, halogen, C₁-C₆ alkyl, and C₁-C₆ haloalkyl;
R₂ is hydrogen, C₁-C₆ alkyl, C₁-C₆ haloalkyl, and C₃-C₆ cycloalkyl;
R₃ is hydrogen, halogen, C₁-C₆ haloalkyl, C₁-C₆ haloalkoxy, or SF₅;
R₄ is hydrogen or methyl;
and in Formula II, X is CH or N.

3. The pharmaceutical composition according to claim 1, wherein R₁ is hydrogen or fluorine.

4. The pharmaceutical composition according to claim 1, wherein R₂ is cyclopropyl or methyl.

5. The pharmaceutical composition according to claim 1, wherein R₃ is CF₃.

6. The pharmaceutical composition according to claim 1, wherein R₄ is hydrogen or methyl.

7. The pharmaceutical composition according to claim 1, wherein X is N.

8. The pharmaceutical composition according to claim 1, wherein, the PRMT5 inhibitor as the first active ingredient is selected from the group consisting of the following compounds and combinations thereof:

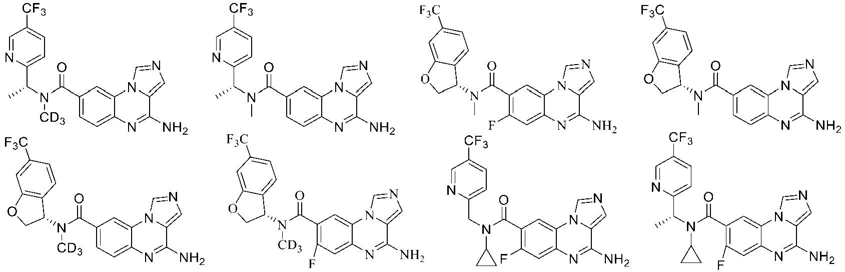

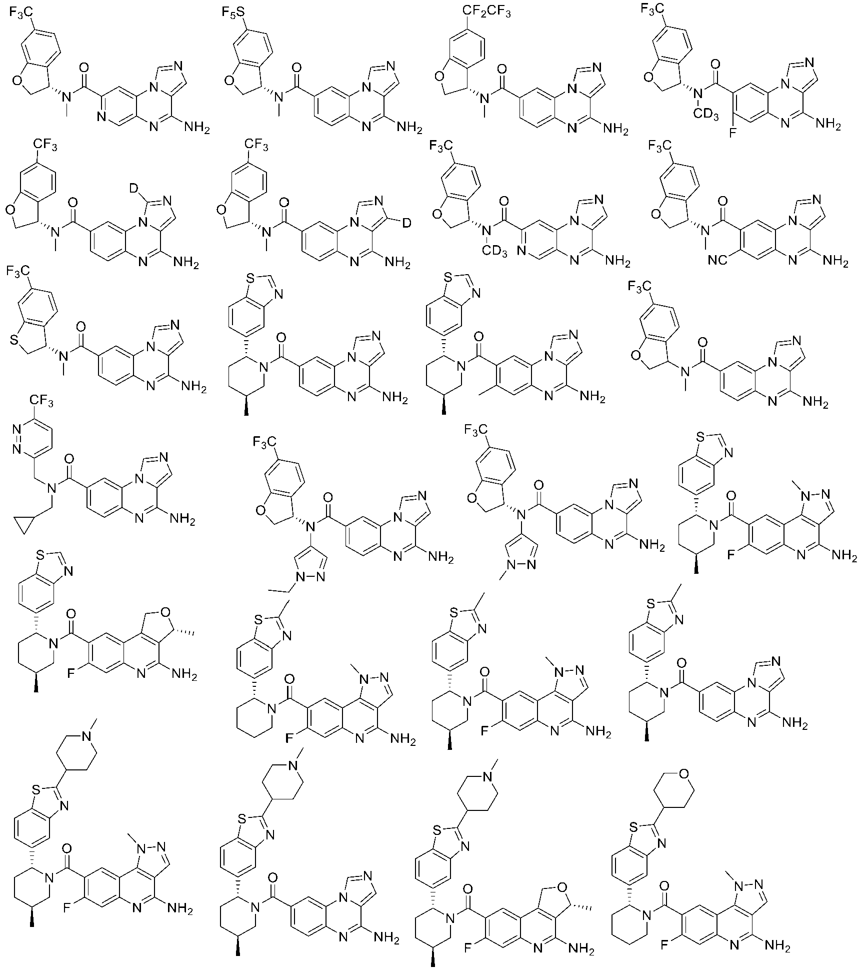

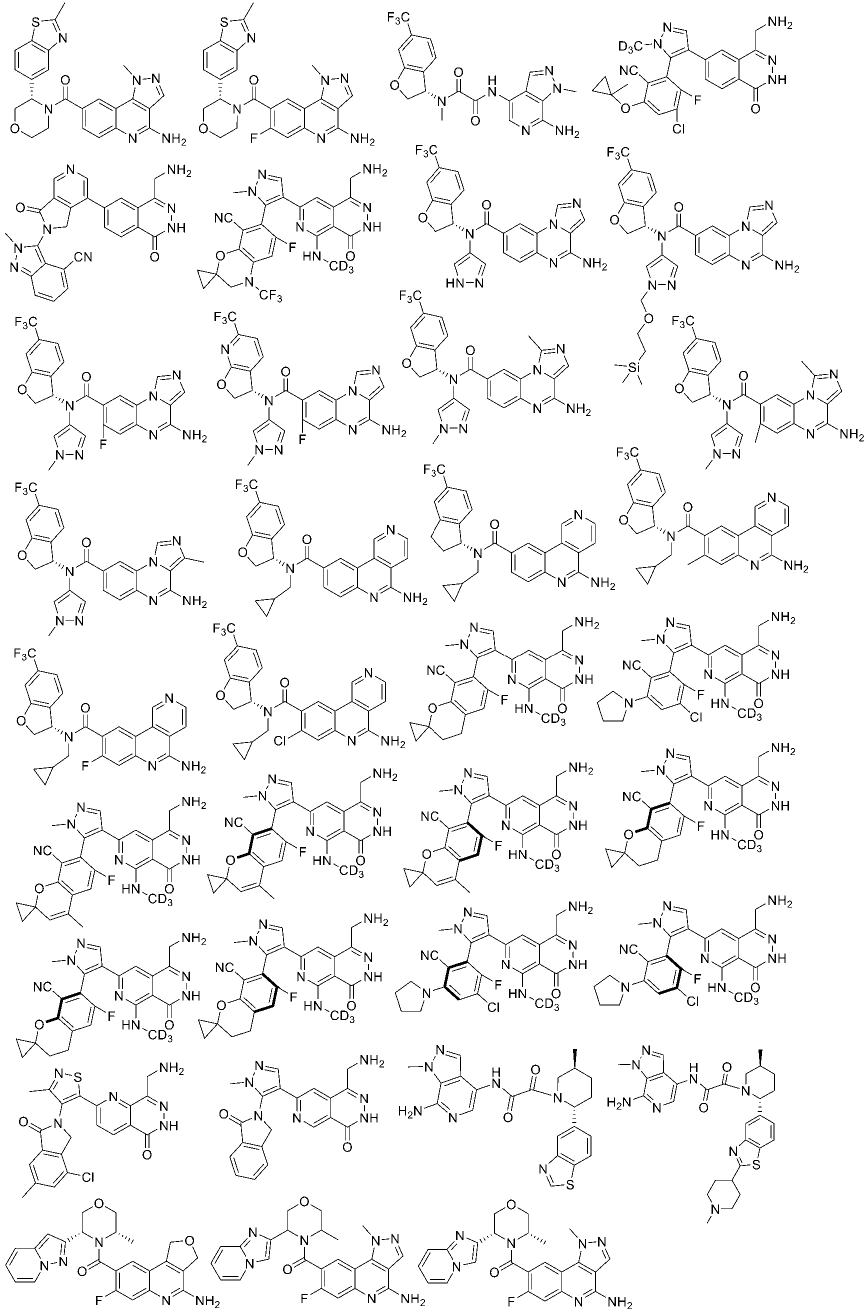

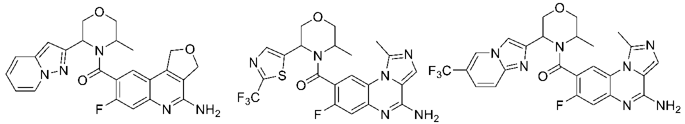

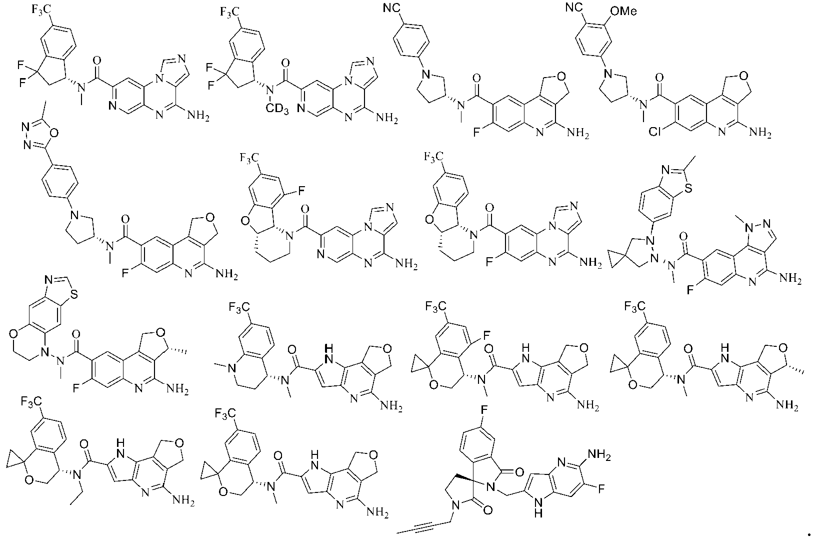

9. The pharmaceutical composition according to any one of claims 1-8, wherein, the EGFR inhibitor as the second active ingredient is selected from any one of the following compounds or pharmaceutically acceptable salts thereof: Osimertinib (Tagrisso^{®}), Almonertinib (Ameile^{®}), Dacomitinib (VIZIMPRO^{®}), Afatinib (Gilotrif^{®}), Icotinib (Conmana^{®}), Erlotinib (Tarceva^{®}), Gefitinib (Iressa^{®}), Zorifertinib (AZD3759), Lazertinib, Nazartinib (EGF816), Rociletinib (CO1686), HM61713, Naquotinib (ASP8273), Mavelertinib (PF-06747775), Abivertinib (avitinib), Alflutinib (AST2818), Olafertinib (CX-101; RX-518), Almonertinib (aumolertinib; HS-10296), Rezivertinib (BPI-7711), Mobocertinib (TAK-788), BLU-945 (Bluprint), BLU-701 (Bluprint), BBT-176 (Bridge Biotherapeutics), TQB3804 (Chiatai Tianqing), BPI-361175 (Betta), QLH11811 (Qilu), HS10375 (Hansoh), H002 (Hongyun Biotech), DAJH-1050766 (Chengdu Diao Jiuhong), BI-4020 (Boehringer Ingelheim), CH7233163 (Chugai Pharmaceutical), JBI-09-063 or any combination thereof.

10. The pharmaceutical composition according to claim 9, wherein, the EGFR inhibitor as the second active ingredient is selected from Trastuzumab (Herceptin^{®}), Pertuzumab (Perjeta^{®}), Cetuximab (Erbitux^{®}), Amivantamab (JNJ-6372) or a pharmaceutically acceptable salt thereof.

11. The pharmaceutical composition according to claim 9, wherein, the EGFR inhibitor as the second active ingredient is selected from Osimertinib (Tagrisso^{®}), Almonertinib (Ameile^{®}), Dacomitinib (VIZIMPRO^{®}), Afatinib (Gilotrif^{®}), Icotinib (Conmana^{®}), Erlotinib (Tarceva^{®}), Gefitinib (Iressa^{®}) or pharmaceutically acceptable salts thereof and Trastuzumab (Herceptin^{®}), Pertuzumab (Perjeta^{®}), Cetuximab (Erbitux^{®}), and Amivantamab (JNJ-6372).

12. A method for treating cancers or tumors, comprising administering the pharmaceutical composition according to any one of claims 1 to 11 to a subject in need thereof.

13. The method according to claim 12, wherein the tumor or cancer is selected from: glioblastoma multiforme, brain cancer, prostate cancer, pancreatic cancer, mantle cell lymphoma, non-Hodgkin's lymphoma and diffuse large B-cell lymphoma, acute myeloid leukemia, acute lymphoblastic leukemia, multiple myeloma, non-small cell lung cancer, small cell lung cancer, breast cancer, triple-negative breast cancer, gastric cancer, colorectal cancer, ovarian cancer, bladder cancer, hepatocellular carcinoma, esophageal cancer, bile duct cancer, mesothelioma, laryngeal cancer, melanoma, malignant peripheral nerve sheath tumor, osteosarcoma, myxoid chondrosarcoma, soft tissue sarcoma, oropharyngeal squamous cell carcinoma, chronic myeloid leukemia, epidermal squamous cell carcinoma, nasopharyngeal carcinoma, neuroblastoma, endometrial cancer, head and neck cancer, and cervical cancer.

14. The method according to claim 13, wherein the tumor is non-small cell lung cancer.

15. The method according to claim 14, wherein the non-small cell lung cancer is Osimertinib-resistant non-small cell lung cancer.

16. The method according to claim 12, wherein the cancer is metastatic cancer.

17. The method according to claim 16, wherein the metastatic cancer is brain metastatic cancer.
